# EUROPEAN PATENT APPLICATION

(11) **EP 4 484 543 A1**
(43) Date of publication of application: **01.01.2025**
(21) Application number: 23181658.8
(22) Date of filing: 27.06.2023
(51) Int. Cl.: C12M 1/12, A61M 39/18, C12M 1/34, C12M 1/36, G01N 27/28, G01N 27/416

(54) **A SYSTEM FOR INSERTION OF A SENSOR INTO A BIOREACTOR**

(71) Applicant: Imec VZW, 3001 Leuven (BE)
(72) Inventor: VAN LANDEGHEM, Nikte, 1050 Brussels (BE); PAEPS, Filip, 3061 Leefdaal (BE)
(74) Representative: AWA Sweden AB

(57) **Abstract**

According to an aspect of the present inventive concept there is provided a system for insertion of a sensor into a bioreactor, said system comprising: an adaptor defining a channel within the adaptor, a first adaptor end configured for being connected to the bioreactor, and a second adaptor end defining an access to the channel; a sensor packaging comprising a sensor carrier, an enclosure, and a sensor packaging end, the sensor packaging end being adapted for forming an sterile connection to the second adaptor end; wherein the sensor and the sensor packaging space are adapted to be sterilized before insertion and before connecting the sensor packaging to the bioreactor, and wherein the system is adapted to be used with the bioreactor exhibiting a sterile inside environment; wherein the sensor packaging end and the second adaptor end are adapted for allowing insertion of the sensor carrier through a connected sensor packaging end and second adaptor end and through the channel of the adaptor into the bioreactor.

## Description

### Technical field

The present description relates to a system for insertion of a sensor into a bioreactor, a bioreactor sensor system and a method for providing a sensor in a bioreactor.

### Background

Bioreactors can be used for many different applications, where an active biological environment is needed. The bioreactor may be for single use or for multiple use. Regardless of type of system, the bioreactor needs to be sterile before the use, to carry a suitable environment for the biological processes. The sterilization process may be optimized for the bioreactor, using gamma radiation, autoclavation or by using steam as a sterilization method.

For measuring relevant features of the ongoing biological processes in the bioreactor, sensors are used. However, at least in current bioreactor systems for multiple use the sensor needs to be sterilized with the bioreactor. Thus, the methods of sterilizing the sensors are limited to those used for sterilizing the bioreactor. This further limits the number of sensors available for use with a bioreactor, since not all sensors may be sterilized using gamma radiation, autoclavation or by using steam.

Thus, there is a need in the art for improvements.

### Summary

An objective of the present description is to provide a system for insertion of a sensor into a bioreactor, providing freedom in use of sterilization method. A further objective is to provide a bioreactor system adapted for high degree of freedom in sterilization. A further objective is to provide a method for providing a sensor in a bioreactor.

This and other objectives of the present inventive concept are at least partly met by the invention as defined in the independent claims. Preferred embodiments are set out in the dependent claims.

According to an aspect, there is provided a system for insertion of a sensor into a bioreactor, said system comprising:
an adaptor being hollow defining a channel within the adaptor, a first adaptor end configured for being connected to the bioreactor and forming a connection between the channel and an interior of the bioreactor, and a second adaptor end defining an access to the channel;
a sensor packaging comprising a sensor carrier carrying the sensor, an enclosure enclosing a sensor packaging space in which the sensor carrier is arranged, and a sensor packaging end defining an access to the sensor packaging space, the sensor packaging end being adapted for forming an sterile connection to the second adaptor end for connecting the sensor packaging space to the channel of the adaptor;
wherein the sensor and the sensor packaging space are adapted to be sterilized before insertion and before connecting the sensor packaging to the bioreactor, and wherein the system is adapted to be used with the bioreactor exhibiting a sterile inside environment;
wherein the sensor packaging end and the second adaptor end are adapted for allowing insertion of the sensor carrier through a connected sensor packaging end and second adaptor end and through the channel of the adaptor into the bioreactor.

Thanks to the adaptor, the sensor packaging may be sterilized before connecting it to the bioreactor, via the adaptor. Thus, the sensor packaging and the sensor may be sterilized in a way suitable for the sensors, while the bioreactor is sterilized in any conventional way. Due to the sterile connections at the first end and at the second adaptor end, the sterility of the sensor carrier and the sensor can be maintained while the sensor is introduced inside the bioreactor.

It should be realized that the system may be used for different bioreactors. Thus, the bioreactor may be a multiple use bioreactor. The bioreactor may be a single use bioreactor.

Further, it should be realized that the adaptor may have different shapes. The adaptor is hollow, such that it defines a channel within the adaptor. The channel is adapted for insertion of the sensor carrier through the adaptor. The first adaptor end is configured for being connected to the bioreactor. The connection forms a connection between the channel within the adaptor and the interior of the bioreactor. After sterilization, the connection is kept sterile, such that any sterile sensor travelling through the adaptor keeps its sterility. The adaptor may be connected to the bioreactor before sterilization and then be sterilized together with the bioreactor.

The adaptor has a second adaptor end configured for defining an access to the channel. Thus, the second adaptor end provides a way of providing a sensor into the bioreactor via the adaptor. Both the second adaptor end and the first adaptor end are configured such that establishing a connection to the adaptor end does not destroy the sterility. Thus, neither of the openings opens the channel of the adaptor to the outside environment.

The adaptor may be configured to provide an interface into the bioreactor facilitating insertion of the sensor into the bioreactor. The adaptor may thus provide an interface allowing a sensor that is separately sterilized to be inserted into the bioreactor. The adaptor may be configured to provide a small-size add-on to an existing bioreactor for providing an improved freedom of insertion of sensors into the bioreactor.

The sensor packaging comprises a sensor carrier carrying a sensor. The sensor may be carried at the tip of the sensor carrier or at any side of the sensor carrier. The sensor carrier may have different geometries. The geometry of the sensor carrier may be adapted to the geometry of the adaptor, such that the hollow part of the adaptor closely encloses the sensor carrier while the sensor carrier is inserted into the adaptor.

It should be realized that in some embodiments active components of a sensor are arranged such that the sensor in itself forms a sensor carrier. Thus, a separate sensor carrier for carrying the sensor may not necessarily be needed.

Further, the sensor packaging comprises an enclosure which encloses the sensor packaging space in which the sensor carrier is arranged. In other words, the enclosure provides a space in which the sensor carrier may be arranged. The enclosure may be arranged surrounding the sensor carrier along the sensor carrier. The enclosure may permit gases to pass through a material of the enclosure. An advantage of this is that gas-based sterilization methods may be used.

The enclosure may further have a handle at a side of the sensor packaging such that the sensor packaging may be easily handled. The handle may further provide a function of fixating the sensor carrier in the sensor packaging.

The sensor packaging further has a sensor packaging end. The sensor packaging end defines an access to the sensor packaging space. The sensor packaging end may be arranged close to the arrangement of the sensor on the sensor carrier, such that when opening the sensor packaging end the sensor may easily be exposed through the sensor packaging end. Further, the sensor packaging end may be closed, such that a sterile environment is kept within the sensor packaging.

In other words, the sensor packaging comprises a sensor carrier carrying a sensor, a sensor packaging defining a sensor space and a sensor packaging end.

The sensor packaging end is adapted for forming a sterile connection to the second adaptor end. Thus, the sensor packaging may be connected to the adaptor via the sensor packaging end and the second adaptor end. By the connection, the sensor packaging space is connected to the channel of the adaptor while maintaining the sterility of the sensor packaging.

The sensor and the sensor packaging space are adapted to be sterilized before insertion and before connecting the sensor packaging to the bioreactor. Thus, the sensor and the sensor packaging may be sterilized in any way suitable for the specific sensor. The system is adapted to be used with the bioreactor exhibiting a sterile inside environment. In other words, the bioreactor and the adaptor are sterilized before the connection to the sterile sensor packaging.

The sensor packaging end and the second adaptor end are adapted for allowing insertion of the sensor carrier through a connected sensor packaging end and second adaptor end and through the channel of the adaptor into the bioreactor. It should be realized that the sensor may be arranged such that only parts of the sensor carrier are inserted into the bioreactor. In other words, only a tip of the sensor carrier or a short part of the sensor carrier may be inserted into the bioreactor.

According to one embodiment the adaptor may be adapted to be screwed to the bioreactor. The connection of the first adaptor end to the bioreactor may be made in several ways. One way is to screw the adaptor on to the bioreactor. The connection may be made using a screw with PG13.5 tread size. This may ensure that the adaptor is adapted to a commonly used interface of the bioreactor.

According to one embodiment the adaptor may be configured to lock the sensor carrier in a certain rotation before allowing insertion of the sensor carrier into the bioreactor. Thus, the adaptor may have a system for locking the orientation of the sensor carrier. Such system may be a tri clamp system, being arranged between the first adaptor end and the second adaptor end, such that the sensor carrier is inserted through the connected sensor packaging end and second adaptor end, before the orientation of the sensor carrier is fixed. Then, the sensor carrier is further inserted through the connected first adaptor end and the bioreactor. An advantage of this is that a sensor being arranged at any side of the sensor carrier may be oriented in a certain way before insertion. An advantage of having the sensor arranged at a side of the sensor is that bubbles at the end of the sensor carrier can be prevented to influence the measurement of the sensor.

According to one embodiment the sensor may be adapted for measuring physical parameters, chemical parameters or biochemical parameters. Physical parameters may for instance be temperature, conductivity and cell density. Chemical parameters may for instance be pH, ions, dissolved gasses such as oxygen. Biochemical parameters may for instance be protein contents, contaminants, metabolites such as glucose.

Further, more than one sensor may be arranged on the sensor carrier, such that more than one parameter may be measured at the same time.

According to one embodiment the adaptor may comprise a first sterile connector at the second adaptor end and the sensor packaging may comprise a second sterile connector at the sensor packaging end. Thus, the sterile connection between the sensor packaging end and the second adaptor end may be formed by sterile connectors arranged at the respective ends. The sterile connectors may be configured to allow being attached without breaking the sterility and may further allow a connection to be formed through the connected ends in an internal environment for which sterility is maintained. Thus, the sensor packaging may be connected to the adaptor while maintaining a sterile inside environment. The sterile connectors may be adapted for the sensor carrier to be inserted through the connected ends.

According to one embodiment the second adaptor end and the sensor packaging end may be adapted for forming the sterile connection by sterile connection welding. This may be an alternative manner for achieving the sterile connection.

According to one embodiment the enclosure may be a collapsible enclosure. Thus, the enclosure may collapse during the insertion of the sensor carrier through a connected sensor packaging end and second adaptor end and through the channel of the tubing of the adaptor into the bioreactor. This may be suitable for allowing control of the sensor carrier while moving the sensor carrier a substantial distance within the sterile environment to insert the sensor into the bioreactor.

According to one embodiment the adaptor may further comprise a closure device configured to enclose the adaptor, at a position between the first adaptor end and the second adaptor end. The enclosure device may be configured such that it can seal the adaptor against the sensor carrier when the sensor carrier is inserted into the bioreactor. An advantage of this, is that any biological material from the bioreactor may be unable to leak into the sensor packaging. This is advantageous since it limits the risk of contamination of the sensor packaging.

According to another aspect, there is provided a bioreactor sensor system, comprising:
a system according to above,
a bioreactor defining an interior supporting a biologically active environment;
wherein the bioreactor is adapted to be sterilized for sterilizing the interior while the adaptor is connected to the bioreactor and before the sensor packaging is connected to the adaptor; and
wherein the sensor and the sensor packaging space are adapted to be sterilized before insertion and before connecting the sensor packaging to the adaptor and the bioreactor.

This aspect may generally present the same or corresponding advantages as the former aspect.

The bioreactor may be a single use bioreactor. The bioreactor may be a multiple use bioreactor.

There may be several advantages with using the present bioreactor sensor system. Any sensor may be used, regardless of the requirement of sterilization method. Thus, the biologically active environment inside the bioreactor may be measured by the sensor in a plurality of ways.

According to a further aspect, there is provided a method for providing a sensor in a bioreactor, said method comprising:
connecting an adaptor to the bioreactor, the adaptor being hollow defining a channel within the adaptor, a first adaptor end being connected to the bioreactor and a second adaptor end defining an access to the channel, wherein the connecting of the adaptor to the bioreactor forms a connection between the channel and an interior of the bioreactor;
connecting a sensor packaging to the adaptor;
   wherein the sensor packaging comprises a sensor carrier carrying the sensor, an enclosure enclosing a sensor packaging space in which the sensor carrier is arranged, and a sensor packaging end defining an access to the sensor packaging space, wherein the connecting of the sensor packaging to the adaptor forms an sterile connection between the sensor packaging end and the second adaptor end for connecting the sensor packaging space to the channel of the adaptor;
wherein the sensor and the sensor packaging space are pre-sterilized before connecting the sensor packaging to the adaptor and wherein the bioreactor exhibits a sterile inside environment;
inserting the sensor carrier through the connected sensor packaging end and second adaptor end and through the channel of the adaptor into the bioreactor.

This aspect may generally present the same or corresponding advantages as the former aspects.

The adaptor is connected to the bioreactor, through the first adaptor end. The connecting of the adaptor to the bioreactor forms a connection between the channel and an interior of the bioreactor. Further, the adaptor is hollow and defines a channel within the adaptor. A second adaptor end is defining an access to the channel.

A sensor packaging is connected to the adaptor, by connecting a sensor packaging end to the second end of the adaptor. The sensor packaging comprises the sensor carrier, the enclosure defining the sensor packaging space and the sensor carrier carrying the sensor.

An advantage of the present method is that the sensor and the sensor packaging space are pre-sterilized before connecting the sensor packaging to the adaptor. Thus, the sterilization may be adapted for the sterilization needs of the sensor before the connection to the adaptor.

The adaptor may be sterilized together with the bioreactor.

The sensor carrier is inserted through the connected sensor packaging end and second adaptor end and through the channel of the adaptor into the bioreactor. It should be realized that the sensor may be arranged on the sensor carrier such that the entire sensor carrier need not be inserted into the bioreactor. Instead, the sensor carrier may be only partly inserted into the bioreactor.

According to one embodiment the method may further comprise re-using the sensor of the sensor packaging for multiple uses of the bioreactor, wherein the method further comprises, after a first use of the bioreactor and the sensor, preparing the bioreactor and the sensor for a second use, said preparing comprising:
removing the sensor packaging from the bioreactor;
closing the sensor packaging end and the second adaptor end;
sterilizing the bioreactor while the adaptor is connected to the bioreactor;
separately sterilizing the sensor packaging system comprising a sensor;
re-connecting the sensor packaging to the adaptor, wherein the re-connecting of the sensor packaging to the adaptor forms a sterile connection between the sensor packaging end and the second adaptor end for re-connecting the sensor packaging space to the channel of the adaptor; and
re-inserting the sensor carrier through the re-connected sensor packaging end and second adaptor end and through the channel of the adaptor into the bioreactor.

Thanks to the use of the adaptor, the sensor packaging and the bioreactor may both be separately sterilized and re-used. In other words, after a first use, the sensor carrier may be pulled out of the bioreactor and the sensor packaging may be sterilized in a suitable way while the bioreactor is separately sterilized. The sensor may be sterilized together with the sensor packaging system, or a new sensor may be mounted on the sensor carrier before re-sterilization. By this, the bioreactor and the sensor packaging may be used several times and new material is not needed.

According to one embodiment the enclosure may collapse during the inserting and/or during the re-inserting. Thus, the enclosure may collapse such that it folds during insertion of the sensor carrier or during re-inserting the sensor carrier.

According to one embodiment the sensor may be pre-sterilized using high temperature, radiation or through chemical sterilization. Thus, the sensor may be sterilized using techniques not suitable for the bioreactor. An advantage of the method is that the sensor is separately sterilized in such a way that is most sufficient for the particular type of sensor.

According to one embodiment the connecting of the sensor packaging to the adaptor may comprise connecting a second sterile connector at the sensor packaging end to a first sterile connector at the second adaptor end.

According to one embodiment the connecting the sensor packaging to adaptor may comprise connecting the sensor packaging end to the second adaptor end through sterile welding of the sensor packaging end and the second adaptor end.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1 is a side view of an embodiment according to the invention.
Fig. 2 is a side view of an embodiment according to the invention.
Fig. 3 is a side view of an embodiment according to the invention.
Fig. 4 is a schematic view of an embodiment according to the invention.

### Detailed description

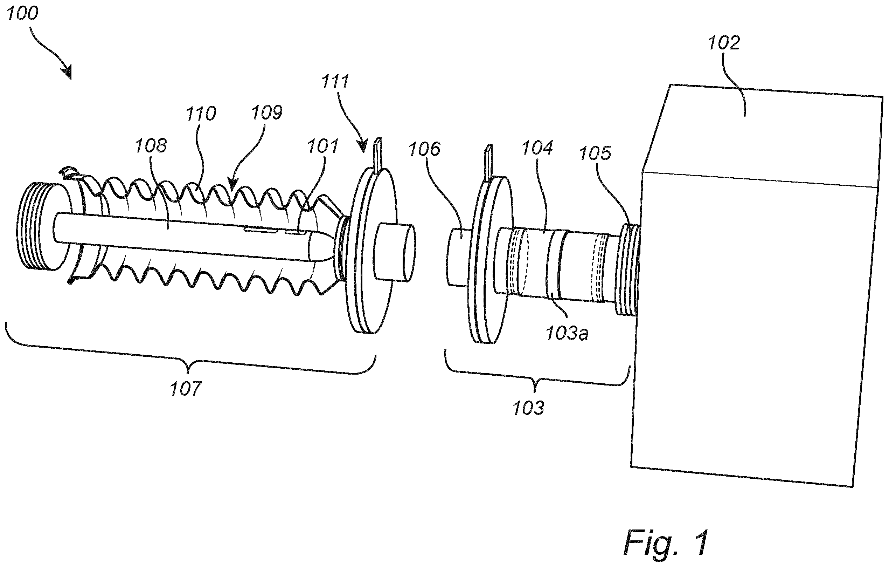

Fig. 1 illustrates a system 100 for insertion of a sensor 101 into a bioreactor 102. The system 100 may be used for different types of bioreactors 102, such as single use bioreactors or multiple use bioreactors.

The system 100 comprises an adaptor 103. The adaptor 103 is hollow, such that it defines a channel 104 within the adaptor 103. It should be realized that the adaptor 103 may have different shapes. The channel of the adaptor 103 is adapted for insertion of a sensor carrier 108 through the adaptor 103 into the bioreactor 102.

The adaptor 103 further comprises a first adaptor end 105 configured for being connected to the bioreactor 102. The connection may be made by screwing the first adaptor end 105 to the bioreactor 102. The connection of the first adaptor end 105 to the bioreactor 102 forms a connection between the channel 104 and an interior of the bioreactor 102.

The adaptor 103 may be sealed to the bioreactor 102 by the use of an O-ring. The O-ring may be arranged in connection with the first adaptor end 105. The O-ring may for instance be arranged such that it is arranged between the first adaptor end 105 and the bioreactor 102, when the first adaptor end 103 is attached to the bioreactor 102.

The adaptor 103 further comprises a second adaptor end 106. The second adaptor end 106 defines an access to the channel 104.

In other words, the first adaptor end 105 and the second adaptor end 106 may be arranged on opposite sides of the adaptor 103 and provides access from the second adaptor end 106 through the channel into the bioreactor 102.

The adaptor 103 may further comprise a closure device 103a. The closure device 103a may be configured to enclose the adaptor, at a position between the first adaptor end 105 and the second adaptor end 106. The closure device 103a may be used to seal the adaptor 103 to the sensor carrier 108, such that biological material from the inside of the bioreactor 102 may not leak into the sensor packaging 107.

The system 100 further comprises a sensor packaging 107 comprising a sensor carrier 108, carrying the sensor 101, an enclosure 109 enclosing a sensor packaging space 110 in which the sensor carrier 108 is arranged and a sensor packaging end 111.

The sensor packaging end 111 is adapted for forming a sterile connection to the second adaptor end 106 for connecting the sensor packaging space 110 to the channel 104 of the adaptor 103.

The adaptor 103 may comprise a first sterile connector at the second adaptor end 106. The sensor packaging 107 may comprise a second sterile connector at the sensor packaging end 111.

The sterile connector at the second adaptor end 106 and at the sensor packaging end 111 may be a type of sterile connector known in the art, such as a CPCO AseptiQuik^{®} Sterile Connector provided by CPC (Colder Products Company). In other words, it may be a sterile connector comprising a membrane preventing the sterile environment to be broken, when the second adaptor end 106 and the sensor packaging end 111 are connected to each other, with sterile connectors adapted for forming a close contact to each other, the membrane may be removed such that the connecting forms an opening through the connected ends.

The second adaptor end 106 and the sensor packaging end 111 may be adapted for forming the sterile connection by sterile connection welding.

The connection of the second adaptor end 106 to the sensor packaging end 111 provides a sterile connection from the sensor packaging 107, through the adaptor 103 and into the bioreactor 102.

The sensor 101 may be adapted for measuring physical parameters, chemical parameters or biochemical parameters. Physical parameters may be temperature, conductivity and cell density. Chemical parameters may be pH, ions, dissolved gasses such as oxygen. Biochemical parameters may be protein contents, contaminants, metabolites such as glucose.

More than one sensor 101 may be arranged on the sensor carrier 108, such that more than one parameter may be measured at the same time.

The sensor 101 may be carried at the tip of the sensor carrier 108 or at any side of the sensor carrier. The sensor carrier 108 may have different geometries. The geometry of the sensor carrier 108 may be adapted to the geometry of the adaptor 103, such that the hollow part of the adaptor 103 closely encloses the sensor carrier 108 while inserted into the adaptor 103.

The sensor 101 and the sensor packaging space 110 are adapted to be sterilized before insertion and before connecting the sensor packaging 107 to the bioreactor 102. The system 100 is adapted to be used with the bioreactor 102 exhibiting a sterile inside environment.

Thus, thanks to the adaptor 103, the sensor packaging 107 may be sterilized before connecting it to the bioreactor 102, via the adaptor 103. In other words, the sensor packaging 107 and the sensor 101 may be sterilized in a way suitable for the sensors, while the bioreactor is sterilized in any conventional way.

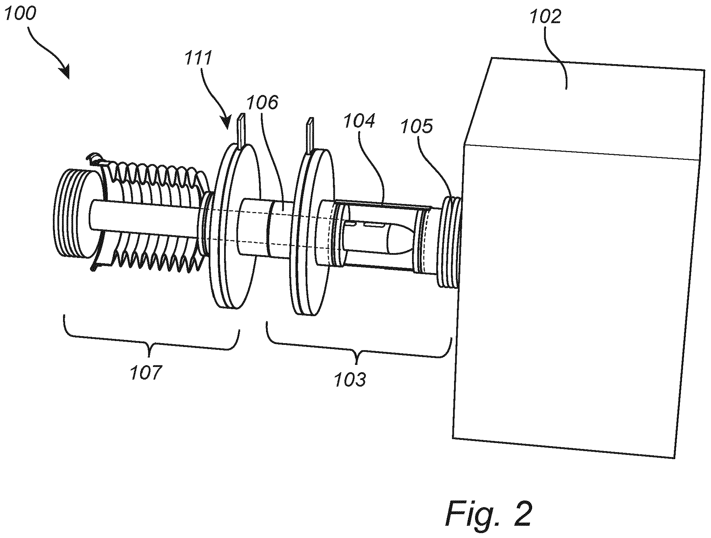

Fig. 2 discloses how the sensor packaging end 111 and the second adaptor end 106 are adapted for allowing insertion of the sensor carrier 108 through a connected sensor packaging end 111 and second adaptor end 106 and through the channel 104 of the adaptor 103 into the bioreactor 102.

The adaptor 103 may be configured to lock the sensor carrier 108 in a certain rotation before allowing insertion of the sensor carrier 108 into the bioreactor 102. Thus, the adaptor 103 may have a system for locking the orientation of the sensor carrier 108. Such system may be a tri clamp system, being arranged between the first adaptor end 105 and the second adaptor end 106, such that the sensor carrier 108 is inserted through the connected sensor packaging end 111 and second adaptor end 106, before the orientation of the sensor carrier 108 is fixed. Then, the sensor carrier 108 is further inserted through the connected first adaptor end 105 and the bioreactor 102.

At insertion, the enclosure 109 may be adapted for collapsing during insertion into the bioreactor 102.

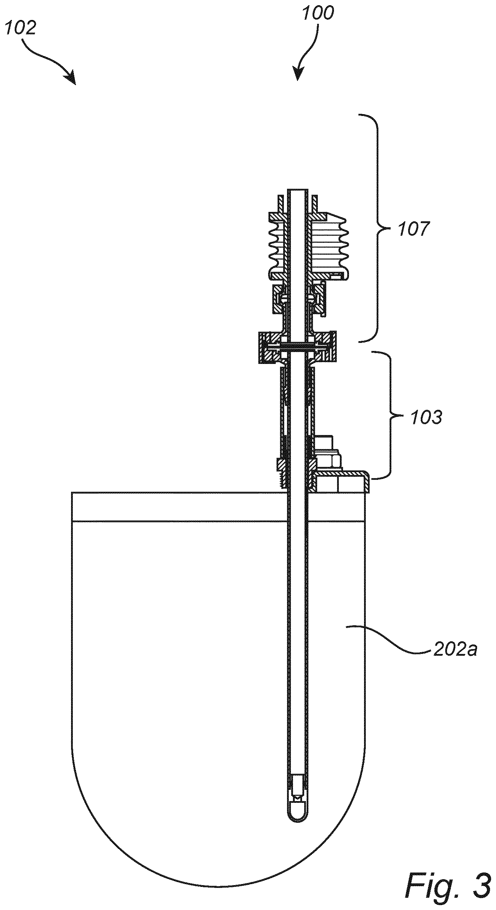

Fig. 3 discloses a bioreactor sensor system 200. The bioreactor system 200 comprises a system 100 according to above.

Further, the bioreactor system 200 comprises a bioreactor 102 defining an interior 202a supporting a biologically active environment. The bioreactor 102 may be adapted for multiple use. The bioreactor 102 may be adapted for single use. The bioreactor 102 is adapted to be sterilized for sterilizing the interior 202a while the adaptor 103 is connected to the bioreactor and before the sensor packaging 107 is connected to the adaptor 103.

The sensor 101 and the sensor packaging space 110 are adapted to be sterilized before insertion and before connecting the sensor packaging 107 to the adaptor 103 and the bioreactor 102.

In a multiple use bioreactor, the sensor must in the prior art be sterilized together with the bioreactor to not influence the sterile environment of the bioreactor negatively. Thanks to the system 100, the sensor may be sterilized before connection and insertion into the bioreactor. This means that the sensor may be sterilized using a method adapted for that specific sensor before it is connected to and inserted in the bioreactor, while the bioreactor may still be sterilized with a method suitable for the bioreactor.

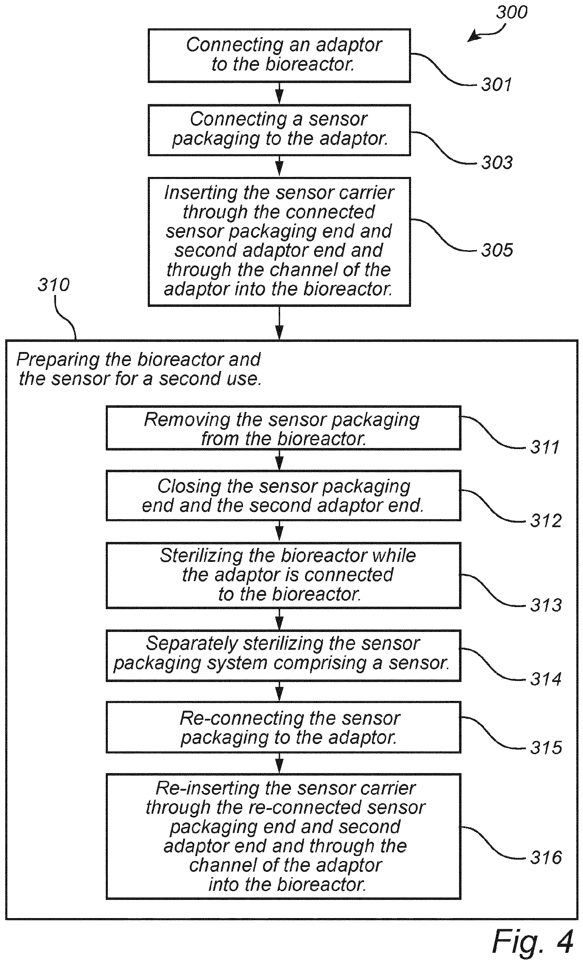

Fig. 4 illustrates a schematic view of a method 300 for providing a sensor 101 in a bioreactor 102.

The method 300 comprises connecting 301 an adaptor 103 to the bioreactor 102. As is disclosed above, the adaptor 103 is hollow defining a channel 104 within the adaptor 103. The adaptor 103 further comprises a first adaptor end 105 being connected to the bioreactor 102 and a second adaptor end 106 defining an access to the channel 104.

The connecting of the adaptor 103 to the bioreactor 102 forms a connection between the channel 104 and an interior 202a of the bioreactor. The adaptor 103 may be sterilized together with the bioreactor 102, such that the connection is sterile.

The method 300 further comprises connecting 303 a sensor packaging 107 to the adaptor 103. By the use of the adaptor 103, the sensor packaging may be sterilized before the connection 303 to the adaptor 103.

As is discussed above, the sensor packaging 107 comprises a sensor carrier 108 carrying the sensor 101, an enclosure 109 enclosing a sensor packaging space 110 in which the sensor carrier 108 is arranged, and a sensor packaging end 111 defining an access to the sensor packaging space 110.

The connecting 303 of the sensor packaging 107 to the adaptor 103 forms a sterile connection between the sensor packaging end 111 and the second adaptor end 106 for connecting the sensor packaging space 110 to the channel 104 of the adaptor 103.

The connection 303 of the sensor packaging 107 to the adaptor 103 may comprise connecting a second sterile connector at the sensor packaging end 111 to a first sterile connector at the second adaptor end 106. For instance, the sterile connectors may each comprise a membrane, which may be removed after connection of the second adaptor end 106 to the sensor packaging end 111. This may allow for the sterility of the sensor packaging 107 and the adaptor 103 to be kept before the connection of the two parts to each other. The removal of the membrane opens up between the connected ends.

The connection 303 of the sensor packaging 107 to the adaptor 103 may comprise connecting the sensor packaging end 111 to the second adaptor end 106 through sterile welding of the sensor packaging end 111 and the second adaptor end 106.

The sensor 101 and the sensor packaging space 110 are pre-sterilized before connecting 303 the sensor packaging 107 to the adaptor 103. The sterilization may for instance be made using high temperature, radiation or through chemical sterilization. Such methods may be suitable for the sensor 101, but may not be possible to use as a sterilization method for the entire bioreactor 102.

The bioreactor exhibits a sterile inside 202a environment. The sterilization of the inside 202a of the bioreactor may be made by any sterilization method, for instance a different sterilization method compared to the sterilization of the sensor 101 and the sensor packaging space 110.

The method 300 further comprises inserting 305 the sensor carrier 108 through the connected sensor packaging end 111 and second adaptor end 106 and through the channel 104 of the adaptor 103 into the bioreactor 102.

During insertion 305, the enclosure 109 may collapse for facilitating moving of the sensor carrier 108 within the sensor packaging space 110 and into the bioreactor.

The method may further comprise re-using the sensor 101 of the sensor packaging 107 for multiple uses of the bioreactor 102.

For this, the method 300 further comprises, after a first use of the bioreactor 102 and the sensor 101, preparing 310 the bioreactor 102 and the sensor for a second use.

The preparing 310 comprises removing 311 the sensor packaging 107 from the bioreactor 102. The removal 310 may be made by disconnecting the sensor packaging end 111 from the second adaptor end 106.

Further, the preparing 310 comprises closing 312 the sensor packaging end 111 and the second adaptor end 106. The preparing 310 may comprise removing the sensor 101 from the sensor carrier 108. If so, a new sensor 101 may be added to the sensor carrier 108 after the sterilization 314.

Further, the preparing 310 comprises sterilizing 313 the bioreactor 102 while the adaptor 103 is connected to the bioreactor 102 and separately 314 sterilizing the sensor packaging system 107 comprising a sensor 101. The sensor 101 may be the already used sensor 101 or it may be a new sensor 101.

The preparing further comprises re-connecting 315 the sensor packaging 107 to the adaptor 103. The re-connecting 315 of the sensor packaging 107 to the adaptor 103 forms a sterile connection between the sensor packaging end 111 and the second adaptor end 106 for re-connecting 315 the sensor packaging space 110 to the channel 104 of the adaptor 103.

Further, the preparing 310 comprises re-inserting 316 the sensor carrier 108 through the re-connected sensor packaging end 111 and second adaptor end 106 and through the channel 104 of the adaptor 103 into the bioreactor 102.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A system (100) for insertion of a sensor (101) into a bioreactor (102), said system (100) comprising:
an adaptor (103) being hollow defining a channel (104) within the adaptor (103), a first adaptor end (105) configured for being connected to the bioreactor (102) and forming a connection between the channel (104) and an interior of the bioreactor (102), and a second adaptor end (106) defining an access to the channel (104);
a sensor packaging (107) comprising a sensor carrier (108) carrying the sensor (101), an enclosure (109) enclosing a sensor packaging space (110) in which the sensor carrier (108) is arranged, and a sensor packaging end (111) defining an access to the sensor packaging space (110), the sensor packaging end (111) being adapted for forming a sterile connection to the second adaptor end (106) for connecting the sensor packaging space (110) to the channel (104) of the adaptor (103);
wherein the sensor (101) and the sensor packaging space (110) are adapted to be sterilized before insertion and before connecting the sensor packaging (107) to the bioreactor (102), and wherein the system (100) is adapted to be used with the bioreactor (102) exhibiting a sterile inside environment;
wherein the sensor packaging end (111) and the second adaptor end (106) are adapted for allowing insertion of the sensor carrier (108) through a connected sensor packaging end (111) and second adaptor end (106) and through the channel (104) of the adaptor into the bioreactor (102).

2. The system (100) according to claim 1, wherein the adaptor (103) is adapted to be screwed to the bioreactor (102).

3. The system (100) according to any one of claims 1 or 2, wherein the adaptor (103) is configured to lock the sensor carrier (108) in a certain rotation.

4. The system (100) according to any one of claims 1 to 3, wherein the sensor (101) is adapted for measuring physical parameters, chemical parameters or biochemical parameters.

5. The system (100) according to any one of claims 1 to 4, wherein the adaptor (103) comprises a first sterile connector at the second adaptor end (106) and the sensor packaging comprises a second sterile connector at the sensor packaging end (111).

6. The system (100) according to any one of claims 1 to 4, wherein the second adaptor end (106) and the sensor packaging end (111) are adapted for forming the sterile connection by sterile connection welding.

7. The system (100) according to any one of claims 1 to 6, wherein the enclosure (109) is a collapsible enclosure.

8. The system (100) according to any one of claims 1 to 7, wherein the adaptor (103) further comprises a closure device (103a) configured to enclose the adaptor, at a position between the first adaptor end (105) and the second adaptor end (106).

9. A bioreactor sensor system (200),comprising:
a system (100) according to any one of the preceding claims,
a bioreactor (102) defining an interior (202a) supporting a biologically active environment;
wherein the bioreactor (102) is adapted to be sterilized for sterilizing the interior (202a) while the adaptor (103) is connected to the bioreactor and before the sensor packaging (107) is connected to the adaptor (103); and
wherein the sensor (101) and the sensor packaging space (110) are adapted to be sterilized before insertion and before connecting the sensor packaging (107) to the adaptor (103) and the bioreactor (102).

10. A method (300) for providing a sensor (101) in a bioreactor (102), said method (300) comprising:
connecting (301) an adaptor (103) to the bioreactor (102), the adaptor (103) being hollow defining a channel (104) within the adaptor (103), a first adaptor end (105) being connected to the bioreactor (102) and a second adaptor end (106) defining an access to the channel (104), wherein the connecting of the adaptor (103) to the bioreactor (102) forms a connection between the channel (104) and an interior (202a) of the bioreactor (102);
connecting (303) a sensor packaging (107) to the adaptor (103);
wherein the sensor packaging (107) comprises a sensor carrier (108) carrying the sensor (101), an enclosure (109) enclosing a sensor packaging space (110) in which the sensor carrier (108) is arranged, and a sensor packaging end (111) defining an access to the sensor packaging space (110), wherein the connecting (303) of the sensor packaging (107) to the adaptor (103) forms an sterile connection between the sensor packaging end (111) and the second adaptor end (106) for connecting the sensor packaging space (110) to the channel (104) of the adaptor (103);
wherein the sensor (101) and the sensor packaging space (110) are pre-sterilized before connecting (303) the sensor packaging (107) to the adaptor (103) and wherein the bioreactor exhibits a sterile inside (202a) environment;
inserting (305) the sensor carrier (108) through the connected sensor packaging end (111) and second adaptor end (106) and through the channel (104) of the adaptor (103) into the bioreactor (102).

11. The method (300) according to claim 10, further comprising re-using the sensor (101) of the sensor packaging (107) for multiple uses of the bioreactor (102), wherein the method (300) further comprises, after a first use of the bioreactor (102) and the sensor (101), preparing (310) the bioreactor (102) and the sensor for a second use, said preparing (310) comprising:
removing (311) the sensor packaging (107) from the bioreactor (102);
closing (312) the sensor packaging end (111) and the second adaptor end (106);
sterilizing (313) the bioreactor (102) while the adaptor (103) is connected to the bioreactor (102);
separately (314) sterilizing the sensor packaging system (107) comprising a sensor (101);
re-connecting (315) the sensor packaging (107) to the adaptor (103), wherein the re-connecting (315) of the sensor packaging (107) to the adaptor (103) forms a sterile connection between the sensor packaging end (111) and the second adaptor end (106) for re-connecting (315) the sensor packaging space (110) to the channel (104) of the adaptor (103); and
re-inserting (316) the sensor carrier (108) through the re-connected sensor packaging end (111) and second adaptor end (106) and through the channel (104) of the adaptor (103) into the bioreactor (102).

12. The method (300) according to anyone of claims 10 or 11, wherein the enclosure (109) collapses during the inserting (305) and/or during the re-inserting (316).

13. The method (300) according to any one of claims 10 to 12, wherein the sensor (101) is pre-sterilized using high temperature, radiation or through chemical sterilization.

14. The method (300) according to any one of claims 10 to 13, wherein the connecting (303) the sensor packaging (107) to the adaptor (103) comprises connecting a second sterile connector at the sensor packaging end (111) to a first sterile connector at the second adaptor end (106).

15. The method (300) according to any one of claims 10 to 14, wherein the connecting (303) the sensor packaging (107) to adaptor (103) comprises connecting the sensor packaging end (111) to the second adaptor end (106) through sterile welding of the sensor packaging end (111) and the second adaptor end (106).
